# EUROPEAN PATENT APPLICATION

(11) **EP 0 545 520 A1**
(43) Date of publication of application: **09.06.1993**
(21) Application number: 92306732.6
(22) Date of filing: 23.07.1992
(51) Int. Cl.: A61L 11/00

(54) **Medical waste sterilizing apparatus**

(30) Priority: 03.12.1991 JP 357466/91
(71) Applicant: SHOEI MANUFACTURING CO., LTD., Osaka 547 (JP)
(72) Inventor: Yoshioka, Yoshiki, Ikoma-district, Nara 639-11 (JP); Ohno, Kazuyuki, Ikoma-district, Nara 639-11 (JP)
(74) Representative: Clifford, Frederick Alan

(57) **Abstract**

Medical waste suitably accumulated in a bag or bags (21,22) is filled via lid (12) into a chamber (2) defined within perforated walls (2a) closed by a hinged bottom plate (13). Hot air at 180 - 200° is passed through the chamber (2) (at 5), exhausted (at 6), and recirculated until the bags (21,22) melt and the contents are sterilized. Then the heater (3) in the air circuit is turned off but cool air is circulated by a blower (7) until the temperature drops to 150°C. At this point a piston/cylinder (14) opens the bottom plate (13) and the waste falls on a rotary member (15). On rotation blades (16) break up the waste against a stationary blade and a drawer (19) accumulates safe sterile pieces of waste for onward disposal.

Advantage is obtained by coating the sterilizing chamber 2 with material which radiates infra-red rays e.g. 4m to 800m at about 180° - 200°C and thereby assists in melting the polymers usually present.

Another embodiment permits of continuous processing using a partitioning wheel indexed to provide successive compartments for loading, heating and cooling, and gravity transfer for breakup on the rotary equipment (15,16). This gives a continuous processing facility. If desired it can also be coated to radiate infra-red rays.

## Description

The present invention relates to medical waste sterilizing apparatus.

Medical waste such as used syringes, or syringe needles, plastic containers, instillators, test tubes and the like must be separately processed from other waste to avoid danger to disposal operatives. On-site incinerators are preferred, but very expensive for the average hospital, because incineration at about 1000°C leads to formation of chlorine gas, NOx and dust, which have to be suppressed, whereby incinerators have to be large and complex.

The present invention therefore sets out to provide means whereby medical waste can be sterilized and brought into a form suitable for onward transport to and handling at a more distant incineration or other ultimate disposal facility, and moreover sets out to provide medical waste sterilizing apparatus of efficient operation, of easy use, and of low running cost.

In one aspect the invention consists in a method of treating medical waste, characterised by heating an accumulation of the waste in circulating air until it is sterilized, (e.g. to 180° - 200°C) thereafter cooling the sterilized waste in a current of air until it is cuttable or breakable (e.g. to 150°C or below), thereafter causing or allowing the waste to pass to cutting or breaking means which reduces it to a safe particulate form optionally accumulating the safe and sterile particulate waste for onward disposal.

In another aspect the invention consists in medical waste sterilizing apparatus characterised by possessing (a) a sterilizing chamber provided with an air inlet and outlet, and connected to a selectively operable air heater and air blower in an air circulation circuit so as to provide as required hot sterilizing air or unheated cooling air and (b) means for breaking up cooled sterilized waste into safe broken-up form, located to be fed from the said sterilizing chamber.

Advantageously the sterilizing chamber is coated at least in part with a coating radiating infra-red radiation at the sterilizing temperature, such as a ceramic or ceramic-based paint radiating infra-red of wavelength from 4m to 800m at a temperature from 180°C to 200°C.

One form of equipment for batch processing comprises a sterilizing chamber with perforate walls, a removable lid and a hinged bottom plate selectively openable to allow the contents to fall under gravity; ducting to allow circulated air to pass through the perforations and sterilizing chamber a bladed rotary break-up equipment beneath the sterilizing chamber whereby cooled sterilized waste can be broken up; and an accumulation chamber beneath the rotary break-up equipment to accumulate safe sterile waste in broken-up form for onward disposal.

Another form of such equipment for more continuous processing possesses (a) a rotary bladed partitioning wheel with a vertical axis of rotation and a plurality of vertical walls (b) a surrounding housing defining with the walls a corresponding plurality of chambers (c) a stationary top plate apertured over at least one of the chamber positions as a loading aperture, the top plate being provided with air inlet means from the air circulation circuit and (d) a stationary bottom plate which is (i) perforated under at least one subsequent chamber position, in the rotation direction, with the perforations providing air outlet means into the air circulation circuit, to define a sterilizing chamber and (ii) apertured at least under one successive chamber position to define a gravity outlet for sterilized and cooled material located above the breakup means: whereby an accumulation of medical waste can be loaded into the top plate aperture; conveyed by rotation of the partitioning wheel over the bottom plate perforations to be heat-sterilized and cooled while providing a new space beneath the loading aperture for a subsequent accumulation of waste; and further conveyed, while providing a further loading chamber, to the aperture in the bottom plate, to fall into the break-up means.

Typically, five vertical walls are provided on the rotary bladed partitioning wheel, and the top plate aperture extends over two chambers; the bottom plate perforations extend beneath the next two chambers in the sense of rotation, and the bottom plate aperture extends beneath the next chamber.

The invention will be further described with reference to the accompanying drawings, in which:
Fig. 1 is a perspective view of the outside of one embodiment of apparatus according to the present invention;
Fig. 2 is a diagrammatic vertical cross-section taken along the apparatus of Fig. 1;
Fig. 3 is a perspective view of the outside of another embodiment of apparatus according to the present invention, with the lid raised and a disposal box opened;
Fig. 4 is a diagrammatic vertical cross-section of the apparatus of Fig. 3 analogously to that of Figure 2;
Fig. 5 is an exploded perspective view of a rotary portion of the apparatus of Fig. 3; and
Fig. 6 is a graph showing the effect of the long wavelength infrared ray radiation coating;

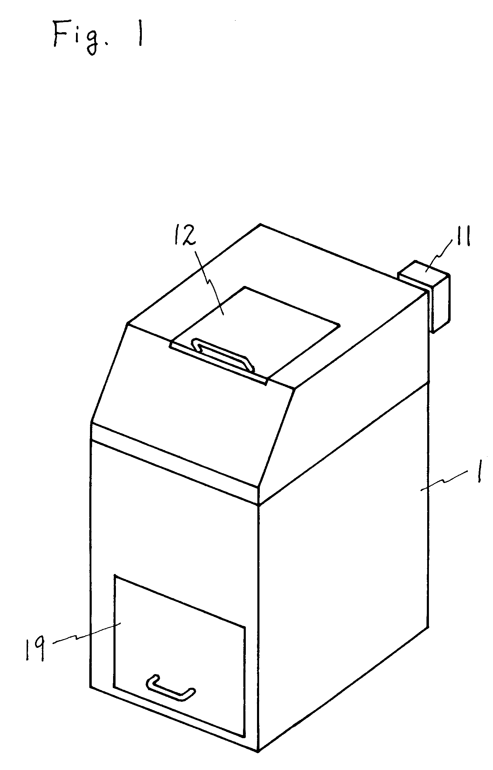

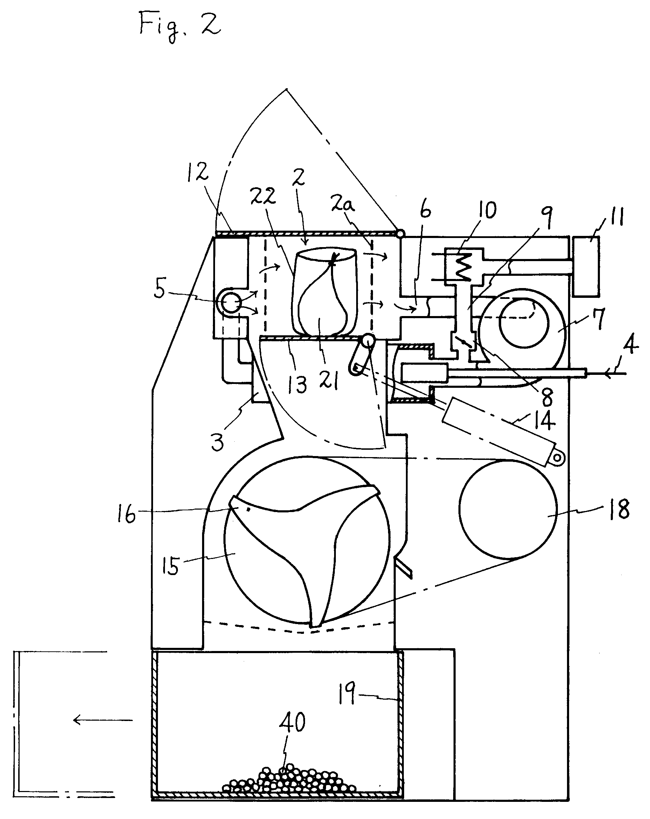

Fig. 1 and Fig. 2 show a first embodiment of the apparatus of the present invention, the apparatus having body 1 with a sterlizing chamber 2 provided in an upper region inside the body.

This sterilizing chamber 2 has a hot air inlet 5 and a hot air outlet 6, and hot air inlet 5 is connected to a heater 3 by means of a suitable duct.

Heater 3 as shown is a burner using a fuel 4 such as oil or gas. An electric heater could also be used, the objective being to heat to about 200°C an air supply set in motion by means of blower 7 which circulates the hot air into the sterilizing chamber 2.

Such hot air circulation improves the heat efficiency of the apparatus and shortens the treatment time.

Air circulation, and exhaust and supply if and as necessary of combustion air and cooling air, can be effected by selective use of blower 7, heater 3, inlet 5 and outlet 6, ducting 9 controlled by damper value 8, and discharge unit 11. Heater 10 is provided in ducting 9 for reasons discussed below.

Sterilizing chamber 2 is defined by a perforate surrounding wall 2a allowing passage of circulated gases; a lid 12; and a pivotable bottom plate 13 connected to a piston/cylinder actuator 14.

Beneath the sterilizing chamber is equipment for breaking up the sterilized and cooled waste into harmlessly small component pieces. This equipment is essentially a rotary member 15 driven by motor 18 and carrying a three-bladed cutter 16 acting against a stationary blade which upon rotation reduces to small dimension any material arriving on to the rotary member 15 from above.

Beneath the rotary member 15 is a collection drawer 19 to receive the broken-up waste 40.

In the construction of the equipment it is important that the perforate walls 2a, a defining the sterilizing chamber should have a coating which radiates long-wavelength infra-red rays when suitably heated at e.g. 180° - 200°C. Examples of such coating are ceramic layers painted or sprayed over suitable base material. The wavelengths produced are typically from 4m to 800m, and we have realised that most synthetic polymers, especially polyester, polyethylene, ABS, or polyvinyl chloride absorb wavelengths of this range very strongly.

The apparatus shown in Figures 1 and 2 operates as follows:-
Medical waste 21 is placed within bag 22 made of synthetic polymeric material of relatively high melting point such as polyester, which does not melt until about 200°C. If desired the waste can be contained in an inner bag 21 made of a different polymer of lower melting point such as polyethylene which melts at about 120°C.

Lid 12 is lifted and the bag 22 (bags 21,22) containing the medical waste are placed in the chamber 2 on the bottom plate 13.

The blower 7 and heater 3 are started, thus circulating and recirculating hot air which passes from inlet 5 through chamber 2 and away through outlet 6 for recirculation. Preferably this is effected at a temperature of from about 180°C to about 200°C, since at temperatures much below 180°C there is risk that sterilization will be incomplete, and at temperatures above 200°C there is an increased possibility of formation of chlorine or other noxious gases, fumes or dust.

The supply of hot (re)circulated gases causes wall 2a to increase in temperature and to radiate in the desired portion of the infra-red spectrum. The bag 22, and any internal bag, disintegrates and the contents are exposed to the high sterilizing temperature within the chamber.

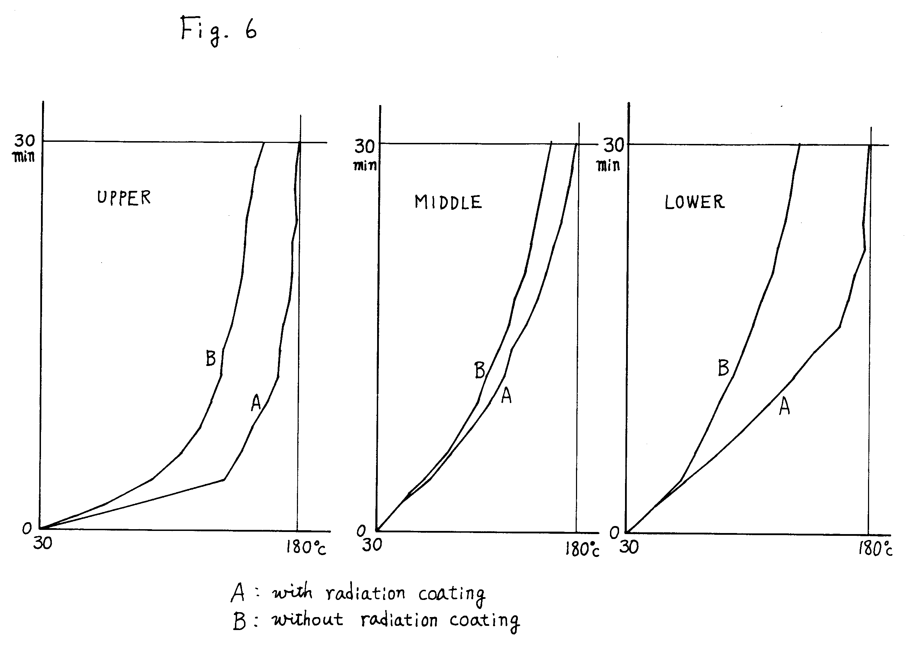

The effect of the infra-red radiation is discussed in more detail below with reference to Fig. 6.

When the hot sterilization has been completed, the heater 3 is switched off and damper 8 is opened. Thus the hot air is purged via discharge line 9, through exhaust unit 11. Heater 10 acts to sterilize any contamination in such air prior to exhaust.

The man skilled in the art will realise that if the heater is a burner (as distinct from an electric heater) there will be some combustion air intake necessary as long as the burner operates. The means to supply this are not shown, but are conventional. Any excess gas produced on combustion can of course be exhausted even during the heating and sterilizing stage, via partly open damper 8 and line 9. If necessary, line 9 could be selectively adaptable to be a combustion air supply.

After a sufficient length of time of the cold air intake cools the sterilized waste 27 to a temperature sufficiently low to resolidify any melted polymeric component parts. Typically, this is about 150°C and can be established by a visible temperature indication, known efflux of time or a sensor directly affecting the next sequential portions of the apparatus. In any event, the rotary member 15 carrying blades 16 is caused or allowed to rotate and piston/cylinder actuator 14 opens the bottom plate to allow sterilized waste to fall by gravity on to the rotary member 15. It then becomes broken up by blades 16 acting against the stationary blade, to form safe broken pieces 40 which drop into container 19. This container is eventually drawn out for onward disposal of the safe and sterile pieces 40.

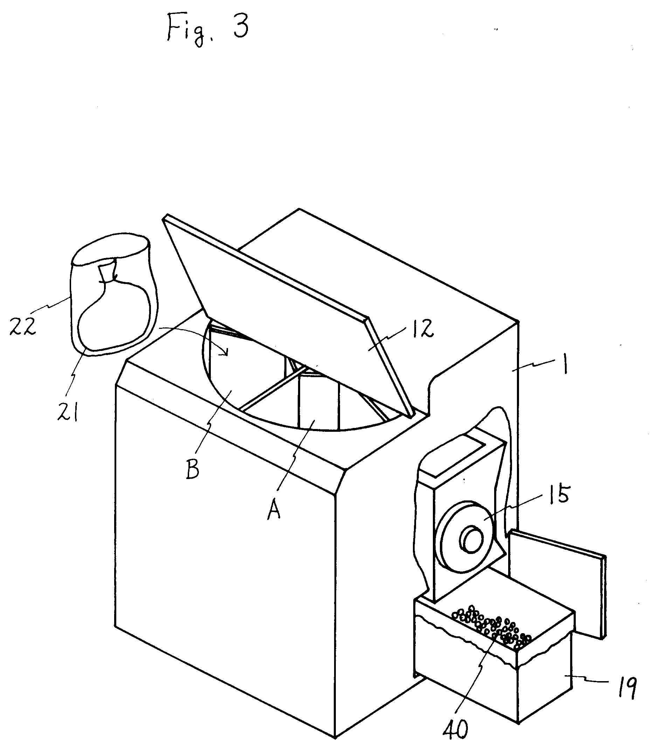

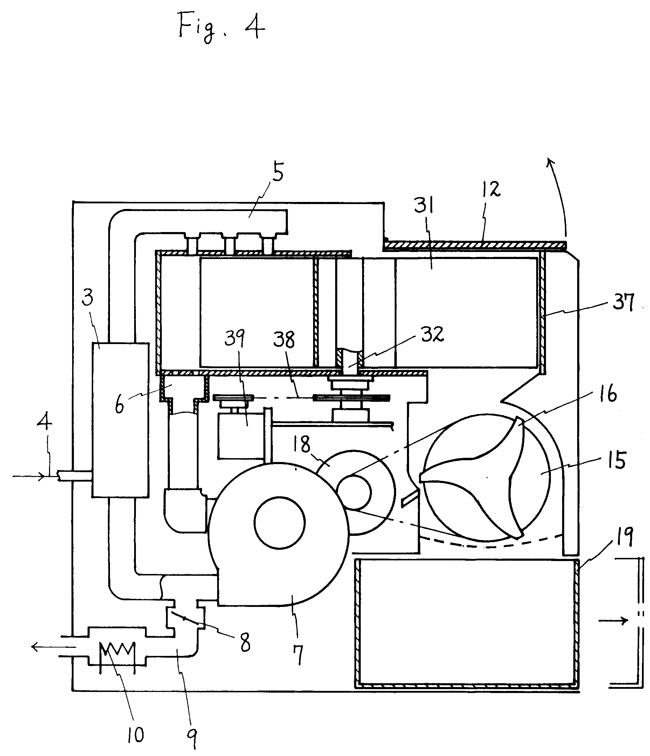

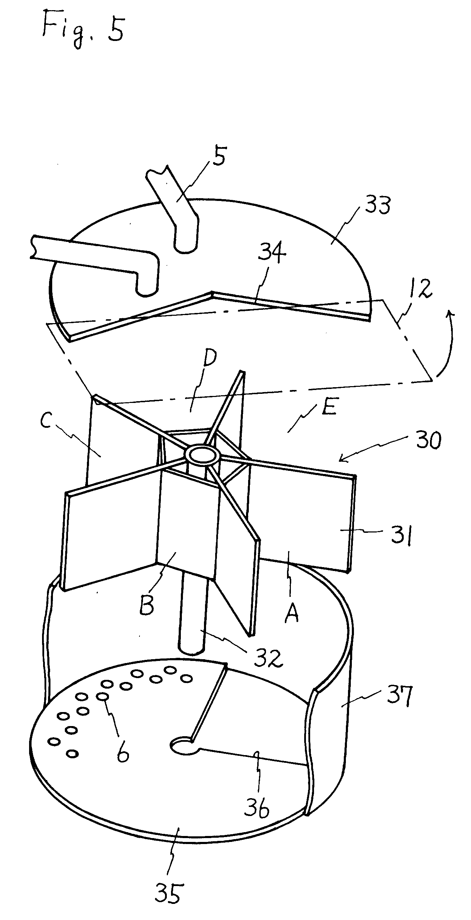

In the embodiment shown in Figs. 3, 4 and 5 the same reference numerals are used where possible to denote the same or closely similar features. Thus, housing 1 has a top lid 12, and a collection drawer 19 for the sterilized particulate waste. It possesses a blower 7, heater 3 and air supply ducting 5 and outlet ducting 6 for a sterilizing chamber. Moreover, gas exhaust arrangements 8, 9, 10 and 11 are as before, and rotary subdivider apparatus 15 and 16 is driven by motor 18 and located over drawer 19 generally as in Figs. 1 and 2.

The difference in the embodiments lies in the sterilizing chamber and associated members.

A rotor 30 comprises a bladed partition wheel 31 subdivided into five compartments A,B,C,D,E and journalled with rotary axis vertical.

In each case, the compartments are further defined by the surrounding wall 37 base plate 35 and top plate 33. Base plate 35 is open only at 36, beneath compartment E, and is perforate beneath compartment D and C, the perforations forming part of the compartment air exhaust ducting 6. Top plate 33 is open only at 34, over compartments A and B, and is provided at C and D with ducting forming part of the air supply ducting 5. Lid 12 can be selectively opened or closed to form a top for of compartment A and B.

Opening 36 is located over the apparatus 15 which breaks the sterilized and cooled product.

The walls of various compartments A-E, as defined by the surrounding member discussed above, can also be coated with the infra-red radiating material as discussed above.

The equipment operates as follows.

Lid 12 is opened and the waste material in a bag 22 (and optionally 21) is placed in each compartment A and B, as described above.

The rotary partition wheel 31 is rotated by a step of 144°. The polymer bags 22 are slid along the bottom plate 35 to overlie the exhaust 6 perforations. The relative temperature resistance of the bags 22 facilitates such sliding.

The blower 7 and heater 3 are started as before, and sterilization, with mechanical break-up of the polymers is effected. Meanwhile, two more such filled bags are to be loaded into those compartments underlying lid 12. On completion of this stage, and again as before, the heater is switched off and cooling air is passed through the two compartments overlying exhaust ducting 6.

When the contents have cooled to below 150°C the rotary partition wheel 31 again rotates through 144°, and the heater again switches on. The second batch of two bags arrives in the sterilizing/cooling position, the previously sterilized and cooled material falls on to rotary equipment 15, and space is provided under lid 12 for two more bags. Essentially, if desired, blower 7 can operate all the time, but heater 3 is intermittent. Motor 18 can be switched on each time as the rotary partitioning wheel rotates, for such a period as may be necessary to break up all of the material.

More or fewer compartments could be used, e.g. three or seven compartments.

It will be appreciated that the embodiment of Figs 1 and 2 is concerned with a single batch feed of medical waste, into a compartment from which sterilized and cooled waste can fall by gravity into break-up equipment prior to onward disposal as safe sterile waste material in subdivided form. This embodiment utilizes both hot air and the effect of the infra-red radiation. The embodiment of Figs. 3, 4 and 5 is concerned with a more continuous process, utilizing continuous feed of bagged accumulations of waste, but again based upon the techniques and procedures of heating, cooling and break-up of the cooled products. In this latter embodiment the use of the infra-red radiating material is optional.

Fig. 6 shows a in graphical form the temperature change in a bag of medical waste, measured respectively in the upper portion, middle portion and lower portion thereof, and measured with (curve A) and without (curve B) the sterilizing chamber coating producing the I.R rays of suitable wavelength. The temperature changes in each case are measured over 30 minutes, and in each the temperature rises appreciably faster with the coating (curve A) than without (curve B).

## Claims

1. A method of treating medical waste, characterised by heating an accumulation of the waste in circulating air until it is sterilized, thereafter cooling the sterilized waste in a current of air until it is cuttable or breakable, and thereafter causing or allowing the waste to pass to cutting or breaking means which reduces it to a safe particulate form.

2. A method as claimed in claim 1 characterised by comprising the further step of accumulating the safe and sterile particulate waste for onward disposal.

3. A method as claimed in claim 2 characterised in that accumulation of waste to be sterilized is heated to a temperature of 180°C - 200°C to sterilize it, cooled to 150°C or below to prepare it for cutting or breaking, allowed to fall by gravity on to rotary cutting or breaking equipment, and allowed to fall as particulate material from said equipment into an accumulation space.

4. Medical waste sterilizing apparatus characterised by possessing (a) a sterilizing chamber (2) provided with an air inlet (5) and outlet (6), and connected to a selectively operable air heater (3) and air blower (7) in an air circulation circuit so as to provide as required hot sterilizing air or unheated cooling air and (b) means (15,16,18) for breaking up cooled sterilized waste into safe broken-up form (40), located to be fed from the said sterilizing chamber.

5. Medical waste sterilizing apparatus as claimed in claim 4 characterized in that the said sterilizing chamber (2) is coated at least in part with a coating radiating infra-red radiation at the sterilizing temperature.

6. Medical waste sterilizing apparatus as claimed in claim 5 in which the said coating is a ceramic or ceramic-based paint radiating infra-red of wavelength from 4m to 800m at a temperature from 180°C to 200°C.

7. Medical waste sterilizing apparatus as claimed in any one of claims 4, 5 or 6 characterised by comprising: a sterilizing chamber (2) with perforate walls (2a), a removable lid (12) and a hinged bottom plate (13) selectively openable (14) to allow the contents to fall under gravity; ducting (5,6) to allow circulated air to pass through the perforations (2a) and sterilizing chamber; (2) a bladed rotary break-up equipment (15,16,18) beneath the sterilizing chamber (2) whereby cooled sterilized waste can be broken up; and an accumulation chamber (19) beneath the rotary break-up equipment to accumulate safe sterile waste (40) in broken-up form for onward disposal.

8. Medical waste sterilizing apparatus as claimed in claim 4, further characterised by possessing (a) a rotary bladed partitioning wheel (30) with a vertical axis of rotation and a plurality of vertical walls (31) (b) a surrounding housing (37) defining with the walls (31) a corresponding plurality of chambers (A,B,C,D,E) (c) a stationary top plate (33) apertured (34) over at least one of the chamber positions as a loading aperture, the top plate being provided with air inlet means (5) from the air circulation circuit and (d) a stationary bottom plate (35) which is (i) perforated (6) under at least one subsequent chamber position, in the rotation direction, with the perforations (6) providing air outlet means into the air circulation circuit, to define a sterilizing chamber and (ii) apertured (36) at least under one successive chamber position to define a gravity outlet for sterilized and cooled material located above the breakup means (15,16,18): whereby an accumulation of medical waste can be loaded into the top plate aperture (34); conveyed by rotation of the partitioning wheel (30) over the bottom plate perforations (6) to be heat-sterilized and cooled while providing a new space beneath the loading aperture (34) for a subsequent accumulation of waste; and further conveyed, while providing a further loading chamber, to the aperture (36) in the bottom plate (35), to fall into the break-up means (15,16,18).

9. Medical waste sterilizing apparatus as claimed in claim 8 characterised in that five vertical walls (31) are provided on the rotary bladed partitioning wheel (30).

10. Medical waste sterilizing apparatus as claimed in claim 9 characterised in that the top plate aperture (34) extend over two chambers; the bottom plate perforations (6) extend beneath the next two chambers in the sense of rotation, and the bottom plate aperture (36) extends beneath the next chamber.

11. Medical waste sterilizing apparatus as claimed in any one of claims 8, 9 or 10 characterised in that the sterilizing chamber position on the top and bottom plates and/or the vertical walls are coated at least in part with a coating radiating infra-red radiation at the sterilizing temperatures.

12. Medical waste sterilizing apparatus as claimed in claim 11 characterised in that said coating is a ceramic or ceramic-based paint radiating infra-red of wavelength from 4m to 800m at a temperature from 180° to 200°C.
